# EUROPÄISCHE PATENTSCHRIFT

(11) **EP 1 828 157 B1**
(45) Veröffentlichungstag und Bekanntmachung des Hinweises auf die Patenterteilung: **03.03.2010**
(21) Anmeldenummer: 05812127.8
(22) Anmeldetag: 03.12.2005
(51) Int. Cl.: C07D 307/60

(54) **VERFAHREN ZUR HERSTELLUNG VON ALKENYLBERNSTEINSÄUREANHYDRIDEN**
METHOD FOR PRODUCING ALKENYL SUCCINIC ANHYDRIDES
PROCEDE DE PRODUCTION D'ANHYDRIDES D'ACIDE SUCCINIQUE D'ALKENYLE

(30) Priorität: 15.12.2004 DE 102004060295
(43) Veröffentlichungstag der Anmeldung: 05.09.2007
(73) Patentinhaber: Clariant Produkte (Deutschland) GmbH, 65929 Frankfurt am Main (DE)
(72) Erfinder: LEINWEBER, Dirk, 65824 Schwalbach (DE); RAU, Tobias, 55130 Mainz (DE)
(74) Vertreter: Mikulecky, Klaus
(86) Internationale Anmeldenummer: PCT/EP2005/012967
(87) Internationale Veröffentlichungsnummer: WO 2006/066720

(56) Entgegenhaltungen:
- WO-A-97/23474
- US-A- 3 412 111
- US-A- 5 021 169
- E. MÜLLER: "Methoden der organischen Chemie (Houben-Weyl), Bd IV/1b" 1975, GEORG-THIEME-VERLAG , STUTTGART , XP002370094 in der Anmeldung erwähnt Seiten 1059-1063 und 1094-1101.

## Beschreibung

Die vorliegende Erfindung betrifft ein Verfahren zur Herstellung von Alkenylbernsteinsäureanhydriden, das sich durch höhere Umsatzgrade, höhere Reinheit der Reaktionsprodukte und durch verminderte Bildung von teerartigen Nebenprodukten auszeichnet sowie die Verwendung der nach diesem Verfahren hergestellten Verbindungen.

Alkenylbernsteinsäureanhydride werden als Ausgangsstoffe einer Vielzahl von chemischen Produkten, insbesondere als Basis für Emulgatoren, Dispergatoren oder Schmiermittel verwendet.

Die Herstellung von Alkenylbernsteinsäureanhydriden erfolgt im Allgemeinen durch Umsetzung eines Alkens mit Maleinsäureanhydrid bei Temperaturen von 150-250°C in der sogenannten "en-Reaktion". Durch die hohe thermische Belastung der Edukte während der Reaktion neigt einerseits das Maleinsäureanhydrid zur Zersetzung und andererseits das verwendete Alken zur Oxidation oder Polymerisation. Durch diese Zersetzungsprozesse bzw. Nebenreaktionen bilden sich während der Reaktion unerwünschte, unlösliche teerartige Nebenprodukte, die einen negativen Einfluss auf den Umsatzgrad haben und zu stark gefärbten, verunreinigten Produkten führen.

Im Stand der Technik ist bereits eine Vielzahl von chemischen Additiven beschrieben, die in der Lage sind, die Neben- bzw. Zersetzungsreaktionen während der en-Reaktion zu unterdrücken.

US-3 412 111 beschreibt die Synthese von Alkenylbernsteinsäureanhydriden unter Verwendung von Antioxidantien. Bevorzugt sind Hydrochinon, 2,2'-Di(p-hydroxyphenyl)-propan und Phenothiazine.

In US-3 476 774 werden sterisch gehinderte, substituierte Phenole beschrieben, die als primäre Antioxidantien während der Herstellung von Alkenylbernsteinsäureanhydriden wirken. Insbesondere wird auf die Verwendung von 4,4'-Methylenbis-(2,6-di-tert-butylphenol) verwiesen.

Ein weiterer Prozess zur Herstellung von Polyalkenylbernsteinsäureanhydriden wird in US-4 883 886 offen gelegt. Dabei wird die Bildung von Nebenprodukten durch 1,3-Dibrom-5,5-dimethylhydantoin unterdrückt.

Saure oder basischen Katalysatoren werden in DE-A-35 45 133 verwendet, um Alkenylbernsteinsäureanhydride von hoher Qualität bei niedriger Temperatur in hohen Ausbeuten zu erhalten. Beispielsweise wirken hier Titanoxid, Siliciumoxid oder Aluminiumoxid.

In US-5 021 169 wird ein Verfahren zur Herstellung von Alkenylbernsteinsäureanhydriden beschrieben, bei dem Produkte mit reduzierten teerartigen Nebenprodukten und verbesserter Farbe dadurch hergestellt werden, dass die en-Reaktion in Anwesenheit eines Tri(orthoalkylphenyl)phosphits sowie gegebenenfalls unter zusätzlicher Verwendung eines sterisch gehinderten phenolischen Antioxidans durchgeführt wird. Die Verwendung von Metalldesaktivatoren wird nicht beschrieben.

Alle bereits beschrieben Verfahren zur Herstellung von Alkenylbernsteinsäureanhydriden lösen die Probleme der Bildung von teerartigen Nebenprodukten sowie die dadurch erniedrigten Ausbeuten und die Bildung von stark gefärbten Produkten nur zum Teil. Dieses liegt darin begründet, dass die unerwünschten Nebenreaktionen wie beispielsweise Oxidation von Alken und/oder Maleinsäureanhydrid bzw. Polymerisation von Alken und/oder Maleinsäureanhydrid nach sehr komplexen noch nicht ins bis ins Letzte geklärten Mechanismen ablaufen, die durch ein einziges Additiv, welches nur in der Lage ist eine dieser Nebenreaktionen zu unterdrücken, nur beschränkt unterdrückt werden können.

Es bestand also die Aufgabe, ein Additiv zu finden, das bei der Herstellung von Alkenylbernsteinsäureanhydriden zur weitgehenden Vermeidung der Bildung von teerartigen Zersetzungsprodukten, hohen Umsatzgraden und hochreinen Produkten führt.
Überraschenderweise konnte dieses Ziel durch die Verwendung einer synergistischen Additiv-Mischung, die aus einem primären Antioxidans, einem sekundären Antioxidans (Peroxid-Zersetzer) und einem Metalldesaktivator besteht, erreicht werden.

Gegenstand der Erfindung ist daher ein Verfahren zur Herstellung von Alkenylbernsteinsäureanhydriden der Formel (1) 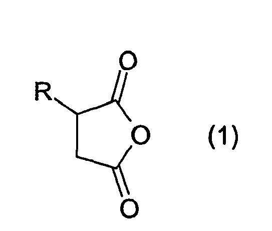 worin
R einen C₄- bis C₂₅₀-Alkylenrest, der linear oder verzweigt sein kann, bedeutet, indem man Maleinsäureanhydrid und ein Alken, welches 4-250 Kohlenstoffatome enthält, bei 150 bis 250°C in Anwesenheit von 0,001 bis 1,0% einer synergistischen Mischung aus einem primären Antioxidans, einem Peroxid-Zersetzer und einem Metalldesaktivator umsetzt.

Das so erhaltene Alkenylbernsteinsäureanhydrid, sowie das korrespondierende, zwangsläufig gebildete Bis-maleinierte Produkt werden dadurch mit hohem Umsatzgrad, hoher Reinheit und verminderter Bildung von teerartigen Nebenprodukten gebildet.

Die erfindungsgemäße Herstellung von Alkenylbernsteinsäureanhydriden mit hohem Umsatzgrad, hoher Reinheit (niedriger Färbung) und verminderter Bildung von teerartigen Nebenprodukten wird grundsätzlich wie im Stand der Technik bekannt durch Umsetzung eines Alkens mit Maleinsäureanhydrid bei hohen Temperaturen (150 bis 250°C) bei einer Reaktionszeit von 5 bis 30 Stunden durchgeführt. Hierzu kann auch ein hochsiedendes, inertes organisches Lösungsmittel verwendet werden. Anstelle von Maleinsäureanhydrid können für das beschriebene Verfahren auch Maleinsäure, Fumarsäure oder deren Ester eingesetzt werden. Die entstehende Verbindung unterscheidet sich dann von der in Formel 1 angegebenen Verbindung durch eine Säure- oder Esterstruktur anstatt der in Formel 1 angegebenen Anhydridstruktur.

Das erfindungsgemäße Verfahren kann bei Normaldruck oder bei erhöhtem Druck durchgeführt werden. Als Alken ist vorzugsweise ein *high reactive* (Gehalt an endständigen Doppelbindungen > 85 %) oder ein *low reactive* Polyisobuten (Gehalt an endständigen Doppelbindungen < 85 %) geeignet.

Um die Oxidation von Alken oder Maleinsäureanhydrid durch Sauerstoffspuren sowie die durch Metallspuren wie z.B. Eisen oder Kupfer induzierte Polymerisation des Alkens oder Zersetzung des Maleinsäureanhydrids zu verhindern, wird im Gegensatz zum Stand der Technik nicht nur *ein* Antioxidans als Inhibitor während der en-Reaktion eingesetzt, sondern eine synergistische Mischung aus einem primären Antioxidans, einem sekundären Antioxidans (Peroxid-Zersetzer) und einem Metalldesaktivator. Hiermit können sämtliche unerwünschten Nebenreaktionen weitgehend unterdrückt werden, was sich in hohen Umsatzgraden, hochreinen und niedrig gefärbten Produkten und einer deutlich verringerten Bildung von teerartigen Nebenprodukten manifestiert.
Die synergistische Mischung aus primärem Antioxidans, sekundärem Antioxidans (Peroxid-Zersetzer) und Metalldesaktivator wird der Eduktmischung aus Alken und Maleinsäureanhydrid in Mengen von 0,001 bis 1,0 Gewichtsprozent (Gew.-%), bevorzugt von 0,05 bis 0,50 Gew.-% und besonders bevorzugt von 0,10 bis 0,30 Gew.-% zugesetzt.

Die synergistische Mischung enthält dabei 1 bis 98 % primäres Antioxidans, 1 bis 98 % sekundäres Antioxidans und 1 bis 98 % Metalldesaktivator, bevorzugt 10 bis 80 % Antioxidans, 10 bis 80 % Peroxid-Zersetzer und 10 bis 80 % Metalldesaktivator, besonders bevorzugt 20 bis 40 % Antioxidans, 20 bis 40 % Peroxid-Zersetzer und 20 bis 40 % Metalldesaktivator. In einer weiteren bevorzugten Ausführungsform addieren sich primäres Antioxidans, Peroxid-Zersetzer und Metalldesaktivator zu 100 Gew.-%.

Geeignete primäre Antioxidantien, sekundäre Antioxidantien und Metalldesaktivatoren sind in Houben-Weyl, Methoden der Organischen Chemie, Band 4/1b (Oxidationen 11), S. 1049-1101, sowie in Ullmann's Encyclopedia of Industrial Chemistry "Antioxidants: Plastics, Additives" als Stabilisatoren für makromolekulare Stoffe beschrieben.

Bevorzugte primäre Antioxidantien in der synergistischen Mischung sind
1. Sterisch gehinderte Trialkylphenole, besonders bevorzugt 2,6-Di-tert.-butyl-4-methylphenol, Bis-(2-hydroxy-5-methyl-3-tert.-butyl-phenyl)methan oder 2,4,6-Tristyrylphenol.
2. Hydrochinon-Derivate, besonders bevorzugt Hydrochinon, 4-tert.-Butoxyphenol oder 2,5-Dihydroxy-1,4-di-tert.-butylbenzol.
3. Aromatische Aminderivate, besonders bevorzugt 1,4-Bis(2-butylamino)-benzol, 4-Isopropylamino-1-phenylamino-benzol oder 4-Butylaminophenol.
4. Aromatische Heterocyclen, besonders bevorzugt Benzimidazol, 2-Mercaptobenz-imidazol oder Phenothiazin.

Bevorzugte sekundäre Antioxidantien in der synergistischen Mischung sind:
1. Trialkylphosphite, wie beispielsweise Tributylphosphit, Trihexylphosphit oder Trioctylphosphit.
2. Triarylphosphite, wie beispielsweise Tris(2,4-di-tert.-butylphenyl)phosphit, Tri(nonylphenyl)phosphite oder Triphenylphosphit.
3. Schwefelhaltige Verbindungen, insbesondere Thioether oder Disulfide, besonders bevorzugt 3,3'-Thiodipropionsäuredistearylester, Distearyldisulfid oder Bis(dimethylaminothiocarbonyl)disulfid.

Bevorzugte Metalldesaktivatoren in der synergistischen Mischung sind:
1. N,N'-Disalicyliden-1,2-diaminopropan, N,N'-Disalicyliden-1,2-diaminoethan oder N,N'-Disalicyliden-1,2-diaminocyclohexan
2. Salicylsäure-Derivate wie Salicylsäure, Acetylsalicylsäure oder Salicylsäureester
3. Hydrazin-Derivate wie Diacylhydrazin, N,N'-Bis(3-methoxy-2-naphthoyl)-hydrazin, N,N'-Bisacetyl(adipinsäuredihydrazid), N,N'-Dibenzaloxalyldihydrazid oder 2',3-Bis-[3-(3,5-di-tert-butyl-4-hydroxyphenyl)-propionyl]propionohydrazid
4. Ethylendiamin-N,N,N',N'-tetraessigsäure (EDTA)
5. Tris[4,4'-thiobis(3-methyl-6-tert.-butylphenol)]phosphit
6. Mannich-artige Reaktionsprodukte aus einem Alkylphenol, einem Aldehyd und einem Polyethylenpolyamin besonders bevorzugt aus Nonylphenol, Formaldehyd und Tetraethylenpentamin.

Im Folgenden wird anhand von Beispielen der Vorteil des erfindungsgemäßen Verfahrens gegenüber den Verfahren nach dem Stand der Technik verdeutlicht.

### Beispiele

### Beispiel 1 (Vergleich)

### Umsetzung von Polyisobuten 950 und Maleinsäureanhydrid (ohne Inhibitor)

In einem 1-Liter Vierhalskolben wurden 475 g (0,50 Mol) Polyisobuten 950 und 63,7 g (0,65 Mol) Maleinsäureanhydrid unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 18 h auf 200°C erhitzt, danach überschüssiges Maleinsäureanhydrid (10,0 g) destillativ entfernt und das Rohprodukt mit 170 g Mineralöl verdünnt. Anschließend wurde das Produkt durch eine Drucknutsche filtriert und der abfiltrierte Rückstand ausgewogen (5,7 g schwarze, teerartige Zersetzungsprodukte). Die Verseifungszahl des dunkelbraunen, trüben Produktes betrug 60 mg KOH/g, der Restolefingehalt 52 % (Mineralöl + unreagiertes Polyisobuten).

### Beispiel 2 (Vergleich)

### Umsetzung von Polyisobuten 950 und Maleinsäureanhydrid mit 0,3 % 2,6-Di-tert.-butyl-4-methylphenol (primäres Antioxidans, Stand der Technik)

In einem 1-Liter Vierhalskolben wurden 475 g (0,50 Mol) Polyisobuten 950, 63,7 g (0,65 Mol) Maleinsäureanhydrid sowie 1,6 g (0,3 Gew.-%) 2,6-Di-tert.-butyl-4-methylphenol unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 18 h auf 200°C erhitzt, danach überschüssiges Maleinsäureanhydrid (6,2 g) destillativ entfernt und das Rohprodukt mit 170 g Mineralöl verdünnt. Anschließend wurde das Produkt durch eine Drucknutsche filtriert und der abfiltrierte Rückstand ausgewogen (3,5 g schwarze, teerartige Zersetzungsprodukte). Die Verseifungszahl des dunkelbraunen, trüben Produktes betrug 65 mg KOH/g, der Restolefingehalt 50 % (Mineralöl + unreagiertes Polyisobuten).

### Beispiel 3 (Vergleich)

### Umsetzung von Polyisobuten 950 und Maleinsäureanhydrid mit 0,3 % Tri(2,4-ditert-butylphenyl)phosphit (sekundäres Antioxidans, Stand der Technik)

In einem 1-Liter Vierhalskolben wurden 475 g (0,50 Mol) Polyisobuten 950, 63,7 g (0,65 Mol) Maleinsäureanhydrid sowie 1,6 g (0,3 Gew.-%) Tri(2,4-di-tert-butylphenyl)phosphit unter Stickstoff-Atmosphäre auf 100°C erwärmt und Sauerstoffspuren durch dreimaliges Evakuieren und Spülen mit Stickstoff bei kräftigem Rühren entfernt. Nun wurde die Reaktionsmischung für 18 h auf 200°C erhitzt, danach überschüssiges Maleinsäureanhydrid (7,9 g) destillativ entfernt und das Rohprodukt mit 170 g Mineralöl verdünnt. Anschließend wurde das Produkt durch eine Drucknutsche filtriert und der abfiltrierte Rückstand ausgewogen (4,3 g schwarze, teerartige Zersetzungsprodukte). Die Verseifungszahl des dunkelbraunen, trüben Produktes betrug 64 mg KOH/g, der Restolefingehalt 50 % (Mineralöl + unreagiertes Polyisobuten).

### Beispiel 4

### Umsetzung von Polyisobuten 950 und Maleinsäureanhydrid mit 0,15 % 2,6-Ditert.-butyl-4-methylphenol und 0,15 % Tri(2,4-di-tert-butylphenyl)phosphit (Mischung aus Inhibitoren der Stand der Technik)

In einem 1-Liter Vierhalskolben wurden 475 g (0,50 Mol) Polyisobuten 950, 63,7 g (0,65 Mol) Maleinsäureanhydrid sowie 0,8 g (0,15 Gew.-%) 2,6-Di-tert.-butyl-4-methylphenol und 0,8 g (0,15 Gew.-%) Tri(2,4-di-tert-butylphenyl)phosphit unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 18 h auf 200°C erhitzt, danach überschüssiges Maleinsäureanhydrid (6,8 g) destillativ entfernt und das Rohprodukt mit 170 g Mineralöl verdünnt. Anschließend wurde das Produkt durch eine Drucknutsche filtriert und der abfiltrierte Rückstand ausgewogen (3,9 g schwarze, teerartige Zersetzungsprodukte). Die Verseifungszahl des dunkelbraunen, trüben Produktes betrug 65 mg KOH/g, der Restolefingehalt 50 % (Mineralöl + unreagiertes Polyisobuten).

### Beispiel 5 (Vergleich)

### Umsetzung von Polyisobuten 950 und Maleinsäureanhydrid mit 0,3% N,N'-Disalicylidene-1,2-diaminopropan (Metalldesaktivator)

In einem 1-Liter Vierhalskolben wurden 475 g (0,50 Mol) Polyisobuten 950, 63,7 g (0,65 Mol) Maleinsäureanhydrid sowie 1,6 g (0,3 Gew.-%) N,N'-Disalicylidene-1,2-diaminopropan unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 18 h auf 200°C erhitzt, danach überschüssiges Maleinsäureanhydrid (8,2 g) destillativ entfernt und das Rohprodukt mit 170 g Mineralöl verdünnt. Anschließend wurde das Produkt durch eine Drucknutsche filtriert und der abfiltrierte Rückstand ausgewogen (5,9 g schwarze, teerartige Zersetzungsprodukte). Die Verseifungszahl des dunkelbraunen, trüben Produktes betrug 63 mg KOH/g, der Restolefingehalt 51 % (Mineralöl + unreagiertes Polyisobuten).

### Beispiel 6

### Umsetzung von Polyisobuten 950 und Maleinsäureanhydrid mit einer synergistischen Mischung aus 0,1 % 2,6-Di-tert.-butyl-4-methylphenol, 0,1 % 3,3'-Thiodipropionsäure-distearylester und 0,05 % N,N'-Disalicylidene-1,2-diaminopropan

In einem 1-Liter Vierhalskolben wurden 475 g (0,50 Mol) Polyisobuten 950, 63,7 g (0,65 Mol) Maleinsäureanhydrid sowie 0,54 g (0,1 Gew.-%) 2,6-Di-tert.-butyl-4-methylphenol, 0,54g (0,1 Gew.-%) 3,3'-Thiodipropionsäure-distearylester und 0,27 g (0,05 Gew.-%) N,N'-Disalicyliden-1,2-diaminopropan unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 18 h auf 200°C erhitzt, danach überschüssiges Maleinsäureanhydrid (4,4 g) destillativ entfernt und das Rohprodukt mit 170 g Mineralöl verdünnt. Anschließend wurde das Produkt durch eine Drucknutsche filtriert und der abfiltrierte Rückstand ausgewogen (0,8 g schwarze, teerartige Zersetzungsprodukte). Die Verseifungszahl des klaren hellgelben Produktes betrug 72 mg KOH/g, der Restolefingehalt 46 % (Mineralöl + unreagiertes Polyisobuten).

### Beispiel 7 (Vergleich)

Umsetzung von Dodecen und Maleinsäureanhydrid mit 0,15 % 2,6-Di-tert.-butyl-4-methylphenol und 0,15 % Tri(2,4-di-tert-butylphenyl)phosphit (Mischung aus Inhibitoren der Stand der Technik)

In einem Autoklaven wurden 1260 g (7,5 Mol) Dodecen, 490 g (5,0 Mol) Maleinsäureanhydrid sowie 2,6 g (0,15 Gew.-%) 2,6-Di-tert.-butyl-4-methylphenol und 2,6 g (0,15 Gew.-%) Tri(2,4-di-tert-butylphenyl)phosphit unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 6 h auf 220°C erhitzt und danach nicht umgesetztes Dodecen und Maleinsäureanhydrid destillativ entfernt. Anschließend wurde das Produkt durch eine Drucknutsche filtriert und der abfiltrierte Rückstand ausgewogen (85 g schwarze, teerartige Zersetzungsprodukte). Die Verseifungszahl des bräunlichen Produktes betrug 395 mg KOH/g.

### Beispiel 8

### Umsetzung von Dodecen und Maleinsäureanhydrid mit 0,1 % 2,6-Di-tert.-butyl-4-methylphenol, 0,1 % 3,3'-Thiodipropionsäure-distearylester und 0,05 % N,N'-Disalicylidene-1,2-diaminopropan

In einem Autoklaven wurden 1260 g (7,5 Mol) Dodecen, 490 g (5,0 Mol) Maleinsäureanhydrid sowie 1,7 g (0,1 Gew.-%) 2,6-Di-tert.-butyl-4-methylphenol und 1,7 g (0,1 Gew.-%) 3,3'-Thiodipropionsäure-distearylester und 0,85 g (0,05 Gew.-%) N,N'-Disalicylidene-1,2-diaminopropan unter Stickstoff-Atmosphäre auf 100°C erwärmt und Sauerstoffspuren durch dreimaliges Evakuieren und Spülen mit Stickstoff bei kräftigem Rühren entfernt.

Nun wurde die Reaktionsmischung für 6 h auf 220°C erhitzt und danach nicht umgesetztes Dodecen und Maleinsäureanhydrid destillativ entfernt. Anschließend wurde das Produkt durch eine Drucknutsche filtriert und der abfiltrierte Rückstand ausgewogen (7,5 g schwarze, teerartige Zersetzungsprodukte). Die Verseifungszahl des hellgelben Produktes betrug 420 mg KOH/g.

Die aufgeführten Beispiele (Tabelle 1) zeigen eindeutig, dass der synergistische Blend aus primärem Antioxidans, sekundärem Antioxidans und Metalldesaktivator (Beispiel 6) den Additiven aus dem Stand der Technik weit überlegen ist. Beispiel 4 belegt zudem, dass eine Zwei-Komponenten-Mischung von Additiven des Standes der Technik dem erfindungsgemäßen synergistischen Blend deutlich unterlegen ist. Ohne die Anwesenheit eines Metalldesaktivators sinken die Umsatzgrade und die Menge an unerwünschten teerartigen Nebenprodukten nimmt zu. Beispiel 5 zeigt weiterhin, dass ein Metalldesaktivator allein ebenfalls nicht in der Lage ist, die Nebenreaktionen zu unterdrücken.

**Tabelle 1:**

| Produkt aus Beispiel | VZ [mg KOH/g] | Restolefingehalt [%] | teerartiges Nebenprodukt [g] | unreagiertes MSA [g] | Aussehen |
|---|---|---|---|---|---|
| 1 | 60 | 52 | 5,7 | 10 | dunkelbraun trüb |
| 2 | 65 | 50 | 3,5 | 6,2 | dunkelbraun trüb |
| 3 | 64 | 50 | 4,3 | 7,9 | dunkelbraun trüb |
| 4 | 65 | 50 | 3,9 | 6,8 | dunkelbraun trüb |
| 5 | 63 | 51 | 5,9 | 8,2 | dunkelbraun trüb |
| 6 | 72 | 46 | 0,8 | 4,4 | hellgelb klar |

Die Beispiele 7 und 8 wurden nicht in Tabelle 1 aufgenommen, da sie nicht wie die Beispiele 1 bis 6 Polyisobutenylbernsteinsäureanhydride betreffen, sondern Dodecenylbernsteinsäureanhydride.

## Patentansprüche

1. Verfahren zur Herstellung von Alkenylbernsteinsäureanhydriden der Formel (1) 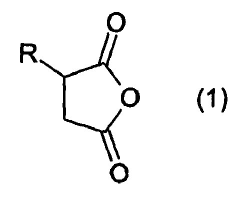 worin
R einen C₄- bis C₂₅₀-Alkylenrest, der linear oder verzweigt sein kann, bedeutet, indem man Maleinsäureanhydrid und ein Alken, welches 4 - 250 Kohlenstoffatome enthält, bei 150 bis 250°C in Anwesenheit einer synergistischen Mischung aus einem primären Antioxidans, einem sekundären Antioxidans und einem Metalldesaktivator umsetzt.

2. Verfahren nach Anspruch 1, wobei als Alkene, Tripropylen, Tetrapropylen, Pentapropylen oder ein C₈₋₃₀-α-Otefin eingesetzt werden.

3. Verfahren nach Anspruch 1 und/oder Anspruch 2, wobei als Alken ein Polyisobuten mit dem Molgewicht 550, 950, 1000, 1300 oder 2300 eingesetzt wird.

4. Verfahren nach einem oder mehreren der Ansprüche 1 bis 3, wobei die synergistische Mischung aus primärem Antioxidans, sekundärem Antioxidans und Metalldesaktivator in einer Menge von 0,001 bis 1,0 Gew.-% eingesetzt wird.

5. Verfahren nach einem oder mehreren der Ansprüche 1. bis 4, wobei die synergistische Mischung 1 bis 98 % primäres Antioxidans, 1 bis 98 % sekundäres Antioxidans und 1 bis 98 % Metalldesaktivator besteht.

6. Verfahren nach einem oder mehreren der Ansprüche 1 bis 5, wobei als primäres Antioxidans in der synergistischen Mischung sterisch gehinderte Trialkylphenole, bevorzugt 2,6-Di-tert.-butyl-4-methylphenol, Bis-(2-hydroxy-5-methyl-3-tert.-butyl-phenyl)methan oder 2,4,6-Tristyrylphenol, eingesetzt werden.

7. Verfahren nach einem oder mehreren der Ansprüche 1 bis 6, wobei als primäres Antioxidans in der synergistischen Mischung ein Hydrochinon-Derivat, bevorzugt Hydrochinon, 4-tert.-Butoxyphenol oder 2,5-Dihydroxy-1,4-di-tert.-butylbenzol, eingesetzt wird.

8. Verfahren nach einem oder mehreren der Ansprüche 1 bis 7, wobei als primäres Antioxidans in der synergistischen Mischung ein aromatisches Aminderivat, bevorzugt 1,4-Bis(2-butylamino)benzol, 4-Isopropylamino-1-phenylamino-benzol oder 4-Butylaminophenol, eingesetzt wird.

9. Verfahren nach einem oder mehreren der Ansprüche 1 bis 8, wobei als primäres Antioxidans in der synergistischen Mischung aromatische Heterocyclen, bevorzugt Benzimidazol, 2-Mercaptobenzimidazol oder Phenothiazin, eingesetzt werden.

10. Verfahren nach einem oder mehreren der Ansprüche 1 bis 9, wobei als sekundäres Antioxidans in der synergistischen Mischung ein Trialkylphosphit, bevorzugt Tributylphosphit, Trihexylphosphit oder Trioctylphosphit, eingesetzt wird.

11. Verfahren nach einem oder mehreren der Ansprüche 1 bis 10, wobei als sekundäres Antioxidans in der synergistischen Mischung ein Triarylphosphit, bevorzugt Tris(2,4-di-tert-butylphenyl)phosphit, Tri(nonylphenyl)phosphit oder Triphenylphosphit, eingesetzt wird.

12. Verfahren nach einem oder mehreren der Ansprüche 1 bis 11, wobei als sekundäres Antioxidans in der synergistischen Mischung schwefelhaltige Verbindungen, bevorzugt 3,3'-Thiodipropionsäuredistearylester, Distearyldisulfid oder Bis(dimethylaminothiocarbonyl)disulfid, eingesetzt werden.

13. Verfahren nach einem oder mehreren der Ansprüche 1 bis 12, wobei als Metalldesaktivator in der synergistischen Mischung N,N'-Disalicyliden-1,2-diaminopropan, N,N'-Disalicyliden-1,2-diaminoethan oder N,N'-Disalicyliden-1,2-diaminocyclohexan eingesetzt wird.

14. Verfahren nach einem oder mehreren der Ansprüche 1 bis 13, wobei als Metalldesaktivator in der synergistischen Mischung Salicylsäure-Derivate wie Salicylsäure, Acetylsalicylsäure oder Salicylsäureester, eingesetzt werden.

15. Verfahren nach einem oder mehreren der Ansprüche 1 bis 14, wobei als Metalldesaktivator in der synergistischen Mischung Diacylhydrazin, N,N'-Bis(3-methoxy-2-naphthoyl)hydrazin, N,N'-Bisacetyl(adipinsäuredihydrazid), N,N'-Dibenzaloxalyldihydrazid oder 2',3-Bis-[3-(3,5-di-tert-butyl-4-hydroxyphenyl)-propionyl]propionohydrazid eingesetzt wird.

16. Verfahren nach einem oder mehreren der Ansprüche 1 bis 15, wobei als Metalldesaktivator in der synergistischen Mischung Ethylendiamin-N,N,N',N'-tetraessigsäure (EDTA) eingesetzt wird.

17. Verfahren nach einem oder mehreren der Ansprüche 1 bis 16, wobei als Metalldesaktivator in der synergistischen Mischung Tris[4,4'-thiobis(3-methyl-6-tert.-butylphenol)]phosphit eingesetzt wird.

18. Verfahren nach einem oder mehreren der Ansprüche 1 bis 17, wobei als Metalldesaktivator in der synergistischen Mischung ein Mannich-artiges Reaktionsprodukt aus einem Alkylphenol, einem Aldehyd und einem Polyethylenpolyamin, bevorzugt aus Nonylphenol, Formaldehyd und Tetraethylenpentamin, verwendet wird.

19. Verfahren nach einem oder mehreren der Ansprüche 1 bis 18, wobei die synergistische Mischung, aus einer primären Antioxdans-Mischung und einer sekundären Antioxidans-Mischung und einer Metalldesaktivatoren-Mischung besteht.

20. Verfahren nach einem oder mehreren der Ansprüche 1 bis 19, wobei das Verfahren unter erhöhtem Druck durchgeführt wird.

21. Verfahren nach einem oder mehreren der Ansprüche 1 bis 20, wobei das eingesetzte Molverhältnis von Alken zu Maleinsäureanhydrid 1 : 0,5 bis 1 : 3 beträgt.

22. Verfahren nach einem oder mehreren der Ansprüche 1 bis 21, wobei die en-Reaktion in einem hochsiedenden, inerten organischen Lösungsmittel durchgeführt wird.

23. Verfahren nach einem oder mehreren der Ansprüche 1 bis 22, wobei es sich bei dem eingesetzten Alken um ein high-reactive und/oder ein low-reactive Polyisobuten handelt.

24. Verfahren nach einem oder mehreren der Ansprüche 1 bis 23, wobei anstelle von Maleinsäureanhydrid Maleinsäure, Fumarsäure oder deren Ester verwendet werden.

## Claims

1. A process for preparing alkenylsuccinic anhydrides of the formula (1) 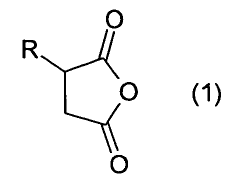 in which
R is a C₄- to C₂₅₀-alkylene radical which may be linear or branched,
by reacting maleic anhydride and an alkene which contains 4-250 carbon atoms at from 150 to 250°C in the presence of a synergistic mixture of a primary antioxidant, a secondary antioxidant and a metal deactivator.

2. The process as claimed in claim 1, wherein the alkenes used are tripropylene, tetrapropylene, pentapropylene or a C₈₋₃₀-α-olefin.

3. The process as claimed in claim 1 and/or claim 2, wherein the alkene used is a polyisobutene of molar mass 550, 950, 1000, 1300 or 2300.

4. The process as claimed in one or more of claims 1 to 3, wherein the synergistic mixture of primary antioxidant, secondary antioxidant and metal deactivator is used in an amount of from 0.001 to 1.0% by weight.

5. The process as claimed in one or more of claims 1 to 4, wherein the synergistic mixture consists from 1 to 98% primary antioxidant, from 1 to 98% secondary antioxidant and from 1 to 98% metal deactivator.

6. The process as claimed in one or more of claims 1 to 5, wherein the primary antioxidant used in the synergistic mixture is a sterically hindered trialkylphenol, preferably 2,6-di-tert-butyl-4-methylphenol, bis(2-hydroxy-5-methyl-3-tert-butylphenyl)methane or 2,4,6-tristyrylphenol.

7. The process as claimed in one or more of claims 1 to 6, wherein the primary antioxidant used in the synergistic mixture is a hydroquinone derivative, preferably hydroquinone, 4-tert-butoxyphenol or 2,5-dihydroxy-1,4-di-tert-butylbenzene.

8. The process as claimed in one or more of claims 1 to 7, wherein the primary antioxidant used in the synergistic mixture is an aromatic amine derivative, preferably 1,4-bis(2-butylamino)benzene, 4-isopropylamino-1-phenylaminobenzene or 4-butylaminophenol.

9. The process as claimed in one or more of claims 1 bis 8, wherein the primary antioxidant used in the synergistic mixture is an aromatic heterocycle, preferably benzimidazole, 2-mercaptobenzimidazole or phenothiazine.

10. The process as claimed in one or more of claims 1 to 9, wherein the secondary antioxidant used in the synergistic mixture is a trialkyl phosphite, preferably tributyl phosphite, trihexyl phosphite or trioctyl phosphite.

11. The process as claimed in one or more of claims 1 to 10, wherein the secondary antioxidant used in the synergistic mixture is a triaryl phosphite, preferably tris(2,4-di-tert-butylphenyl) phosphite, tri(nonylphenyl) phosphite or triphenyl phosphite.

12. The process as claimed in one or more of claims 1 to 11, wherein the secondary antioxidant used in the synergistic mixture is a sulfur compound, preferably distearyl 3,3'-thiodipropionate, distearyl disulfide or bis(dimethylaminothiocarbonyl) disulfide.

13. The process as claimed in one or more of claims 1 to 12, wherein the metal deactivator used in the synergistic mixture is N,N'-disalicylidene-1,2-diaminopropane, N,N'-disalicylidene-1,2-diaminoethane or N,N'-disalicylidene-1,2-diaminocyclohexane.

14. The process as claimed in one or more of claims 1 to 13, wherein the metal deactivator used in the synergistic mixture is a salicylic acid derivative such as salicylic acid, acetylsalicylic acid or salicylic esters.

15. The process as claimed in one or more of claims 1 to 14, wherein the metal deactivator used in the synergistic mixture is diacylhydrazine, N,N'-bis(3-methoxy-2-naphthoyl)hydrazine, N,N'-bisacetyl(adipic hydrazide), N,N'-dibenzaloxalyl dihydrazide or 2',3-bis[3-(3,5-di-tert-butyl-4-hydroxyphenyl)propionyl]propionohydrazide.

16. The process as claimed in one or more of claims 1 to 15, wherein the metal deactivator used in the synergistic mixture is ethylenediamine-N,N,N',N'-tetraacetic acid (EDTA).

17. The process as claimed in one or more of claims 1 to 16, wherein the metal deactivator used in the synergistic mixture is tris[4,4'-thiobis(3-methyl-6-tertbutylphenol)] phosphite.

18. The process as claimed in one or more of claims 1 to 17, wherein the metal deactivator used in the synergistic mixture is a Mannich-type reaction product formed from an alkylphenol, an aldehyde and a polyethylenepolyamine, preferably from nonylphenol, formaldehyde and tetraethylenepentamine.

19. The process as claimed in one or more of claims 1 to 18, wherein the synergistic mixture consists of a primary antioxidant mixture and a secondary antioxidant mixture and a metal deactivator mixture.

20. The process as claimed in one or more of claims 1 to 19, wherein the process is performed under elevated pressure.

21. The process as claimed in one or more of claims 1 to 20, wherein the molar ratio of alkene to maleic anhydride used is from 1:0.5 to 1:3.

22. The process as claimed in one or more of claims 1 to 21, wherein the ene reaction is performed in a high-boiling inert organic solvent.

23. The process as claimed in one or more of claims 1 to 22, wherein the alkene used is a high-reactive and/or low-reactive polyisobutene.

24. The process as claimed in one or more of claims 1 to 23, wherein maleic acid, fumaric acid or esters thereof are used instead of maleic anhydride.

## Revendications

1. Procédé pour la préparation d'anhydrides de l'acide alcénylsuccinique de formule (1) 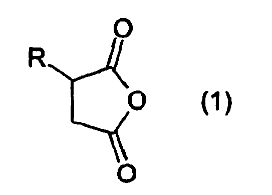 où
R signifie un radical C₄-C₂₅₀-alkylène qui peut être linéaire ou ramifié, en ce qu'on transforme de l'anhydride de l'acide maléique et un alcène, qui contient 4-250 atomes de carbone, à 150 jusqu'à 250°C en présence d'un mélange synergique d'un antioxydant primaire, d'un antioxydant secondaire et d'un désactivateur de métaux.

2. Procédé selon la revendication 1, où on utilise comme alcènes le tripropylène, le tétrapropylène, le pentapropylène ou une α-oléfine en C₈₋₃₀.

3. Procédé selon la revendication 1 et/ou la revendication 2, où on utilise comme alcène un polyisobutène présentant un poids moléculaire de 550, 950, 1000, 1300 ou 2300.

4. Procédé selon l'une ou plusieurs des revendications 1 à 3, où le mélange synergique constitué par un antioxydant primaire, un antioxydant secondaire et un désactivateur de métaux est utilisé en une quantité de 0,001 à 1,0% en poids.

5. Procédé selon l'une ou plusieurs des revendications 1 à 4, où le mélange synergique est constitué par 1 à 98% d'antioxydant primaire, 1 à 98% en poids d'antioxydant secondaire et 1 à 98% en poids de désactivateur de métaux.

6. Procédé selon l'une ou plusieurs des revendications 1 à 5, où on utilise comme antioxydant primaire dans le mélange synergique des trialkylphénols stériquement encombrés, de préférence le 2,6-di-tert-butyl-4-méthylphénol, le bis-(2-hydroxy-5-méthyl-3-tert-butylphényl)méthane ou le 2,4,6-tristyrylphénol.

7. Procédé selon l'une ou plusieurs des revendications 1 à 6, où on utilise comme antioxydant primaire dans le mélange synergique un dérivé d'hydroquinone, de préférence l'hydroquinone, le 4-tert-butoxyphénol ou le 2,5-dihydroxy-1,4-di-tert-butylbenzène.

8. Procédé selon l'une ou plusieurs des revendications 1 à 7, où on utilise comme antioxydant primaire dans le mélange synergique un dérivé aromatique d'amine, de préférence le 1,4-bis(2-butylamino)benzène, le 4-isopropylamino-1-phénylaminobenzène ou le 4-butylaminophénol.

9. Procédé selon l'une ou plusieurs des revendications 1 à 8, où on utilise comme antioxydant primaire dans le mélange synergique des hétérocycles aromatiques, de préférence le benzimidazole, le 2-mercaptobenzimidazole ou la phénothiazine.

10. Procédé selon l'une ou plusieurs des revendications 1 à 9, où on utilise comme antioxydant secondaire dans le mélange synergique un trialkylphosphite, de préférence le tributylphosphite, le trihexylphosphite ou le trioctylphosphite.

11. Procédé selon l'une ou plusieurs des revendications 1 à 10, où on utilise comme antioxydant secondaire dans le mélange synergique un triarylphosphite, de préférence le tris(2,4-di-tert-butylphényl)phosphite, le tri(nonylphényl)phosphite ou le triphénylphosphite.

12. Procédé selon l'une ou plusieurs des revendications 1 à 11, où on utilise comme antioxydant secondaire dans le mélange synergique des composés soufrés, de préférence l'ester distéarylique de l'acide 3,3'-thiodipropionique, le disulfure de distéaryle ou le disulfure de bis(diméthylaminothiocarbonyle).

13. Procédé selon l'une ou plusieurs des revendications 1 à 12, où on utilise comme désactivateur de métaux dans le mélange synergique le N,N'-disalicylidène-1,2-diaminopropane, le N,N'-disalicylidène-1,2-diaminoéthane ou le N,N'-disalicylidène-1,2-diaminocyclohexane.

14. Procédé selon l'une ou plusieurs des revendications 1 à 13, où on utilise comme désactivateur de métaux dans le mélange synergique des dérivés de l'acide salicylique, tels que l'acide salicylique, l'acide acétylsalicylique ou les esters de l'acide salicylique.

15. Procédé selon l'une ou plusieurs des revendications 1 à 14, où on utilise comme désactivateur de métaux dans le mélange synergique la diacylhydrazine, la N,N'-bis(3-méthoxy-2-naphtoyl)hydrazine, le N,N'-bisacétyl(dihydrazide de l'acide adipique), le dihydrazide de N,N'-dibenzaloxalyle ou le 2',3-bis-[3-(3,5-di-tert-butyl-4-hydroxyphényl)-propionyl]propionohydrazide.

16. Procédé selon l'une ou plusieurs des revendications 1 à 15, où on utilise comme désactivateur de métaux dans le mélange synergique l'acide éthylènediamine-N,N,N',N'-tétraacétique (EDTA).

17. Procédé selon l'une ou plusieurs des revendications 1 à 16, où on utilise comme désactivateur de métaux dans le mélange synergique le tris[4,4'-thiobis(3-méthyl-6-tert-butylphénol)]phosphite.

18. Procédé selon l'une ou plusieurs des revendications 1 à 17, où on utilise comme désactivateur de métaux dans le mélange synergique un produit de réaction de type Mannich d'un alkylphénol, d'un aldéhyde et d'une polyéthylènepolyamine, de préférence de nonylphénol, de formaldéhyde et de tétraéthylènepentamine.

19. Procédé selon l'une ou plusieurs des revendications 1 à 18, où le mélange synergique est constitué par un mélange d'antioxydants primaires et d'un mélange d'antioxydants secondaires et d'un mélange de désactivateurs de métaux.

20. Procédé selon l'une ou plusieurs des revendications 1 à 19, où le procédé est réalisé sous pression élevée.

21. Procédé selon l'une ou plusieurs des revendications 1 à 20, où le rapport molaire utilisé d'alcène à anhydride de l'acide maléique est de 1:0,5 à 1:3.

22. Procédé selon l'une ou plusieurs des revendications 1 à 21, où la réaction ène est réalisée dans un solvant organique inerte à point d'ébullition élevé.

23. Procédé selon l'une ou plusieurs des revendications 1 à 22, où il s'agit pour l'alcène utilisé d'un polyisobutène hautement réactif et/ou faiblement réactif.

24. Procédé selon l'une ou plusieurs des revendications 1 à 23, où on utilise au lieu de l'anhydride de l'acide maléique de l'acide maléique, de l'acide fumarique ou leurs esters.
